(19) Europäisches Patentamt
European Patent Office
Office européen des brevets

(11) Numéro de publication : **0 287 430 B1**

(12) # FASCICULE DE BREVET EUROPEEN

(45) Date de publication du fascicule du brevet :
**11.12.91 Bulletin 91/50**

(51) Int. Cl.$^5$ : **A61F 11/04**

(21) Numéro de dépôt : **88400819.4**

(22) Date de dépôt : **05.04.88**

(54) **Prothèse et électrode pour la stimulation électrique de l'oreille interne, et procédé de fabrication de cette électrode.**

(30) Priorité : **06.04.87 FR 8704832**

(43) Date de publication de la demande :
**19.10.88 Bulletin 88/42**

(45) Mention de la délivrance du brevet :
**11.12.91 Bulletin 91/50**

(84) Etats contractants désignés :
**BE CH DE ES FR GB GR IT LI NL SE**

(56) Documents cités :
**EP-A- 0 002 068**
**FR-A- 2 465 474**
**US-A- 4 606 329**

(73) Titulaire : **ASSISTANCE PUBLIQUE**
**3, avenue Victoria**
**F-75100 Paris (FR)**
Titulaire : **AUDIT S.A.**
**96 avenue de la Victoire**
**F-75009 Paris (FR)**

(72) Inventeur : **Frachet,Bruno**
**3 rue de Turin**
**F-95160 Montmorency (FR)**
Inventeur : **David,Michel-Yves**
**120 boulevard du Montparnasse**
**F-75014 Paris (FR)**

(74) Mandataire : **Le Guen, Gérard et al**
**CABINET LAVOIX 2, place d'Estienne d'Orves**
**F-75441 Paris Cédex 09 (FR)**

## Description

La présente invention concerne une prothèse permettant à la fois une stimulation acoustique et une stimulation électrique de l'oreille interne, dans le cas de surdité neuro-sensorielle complète.

On a déjà proposé un certain nombre de techniques pour produire des stimulations électriques de l'oreille interne et notamment des techniques dites "membraneuses" dans lesquelles on applique une électrode sur la fenêtre ronde de l'oreille interne.

La fixation d'une telle électrode est assurée par différents procédés utilisant notamment une colle biologique, un produit spongieux résorbable, des fragments de tissus musculaires. Cette fixation n'est jamais exempte de déplacement de sorte qu'il y a un risque important d'inefficacité du dispositif implanté.

Il faut ajouter que les variations anatomiques de la fenêtre ronde imposent souvent des adaptations de la technique chirurgicale.

La présente invention vise à fournir une prothèse auditive capable de stimuler l'oreille interne par la fenêtre ovale.

La présente invention vise en outre dans un cas particulier à permettre la transformation d'une prothèse classique stapédo-vestibulaire par la fixation à l'extrémité de cette prothèse d'une électrode. On rappelera que les prothèses stapédo-vestibulaires sont utilisées dans le cas de surdité de transmission liée notamment à une otospongiose où l'on remplace l'étrier par un élément d'interposition dont l'une des extrémités est fixée à l'enclume et dont l'autre extrémité est appliquée contre la fenêtre ovale.

La présente invention a ainsi pour objet une prothèse permettant à la fois une stimulation acoutique et une stimulation électrique de l'oreille interne, caractérisée en ce qu'elle comprend un élément d'interposition dont l'une des extrémités est munie de moyens de fixation à la branche descendante de l'enclume et dont l'autre extrémité, destinée à être appliquée sur la fenêtre ovale, est munie d'une surface d'extrémité conductrice constituant une électrode, qui est reliée par un conducteur recouvert d'un revêtement isolant biocompatible à une source de stimulation électrique.

Dans une forme préférée de réalisation de l'invention, l'électrode est constituée par une cupule métallique dans laquelle l'extrémité de l'élément d'interposition est insérée et dont la surface périphérique est recouverte d'un revêtement isolant biocompatible, ladite cupule étant soudée audit conducteur.

La présente invention a également pour objet une électrode de stimulation électrique de l'oreille interne destinée notamment à être appliquée à l'une des extrémités d'une prothèse classique stapédo-vestibulaire, cette électrode étant constituée par une cupule dont la surface périphérique est recouverte d'un revêtement isolant biocompatible, ladite cupule étant soudée à un conducteur recouvert d'un revêtement isolant biocompatible.

Il est à noter que dans les surdités neuro-sensorielles habituellement le système tympano-ossiculaire, ainsi que le système mécanique de l'oreille interne sont normaux. Il est donc possible d'obtenir grâce à la prothèse selon l'invention à la fois une stimulation acoustique déclenchant des phénomènes mécaniques habituels dans l'oreille interne et une stimulation électriques des extrémités nerveuses. Dans ce cas la vibration acoustique n'est pas perçue comme un son. Pour renforcer cette stimulation mécanique on peut utiliser une prothèse auditive conventionnelle disposée dans le conduit auditif externe.

La présente invention a également pour objet un procédé de fabrication d'une électrode en forme de cupule telle que définie précédemment. Selon ce procédé.

a) on fabrique une matrice métallique du diamètre de l'élément d'interposition sur lequel l'électrode sera fixée,

b) on recouvre par un isolant la matière à l'exception d'une zone d'extrémité,

c) on applique contre la zone d'extrémité non recouverte de la matrice l'extrémité d'un fil de métal biocompatible et non oxydable,

d) on forme électrolytiquement un dépôt de métal biocompatible et non oxydable sur la zone non recouverte de la matrice de façon à former une cupule de métal biocompatible et non oxydable lié à l'extrémité du fil de métal biocompatible et non oxydable,

e) on élimine la matrice métallique par voie chimique et

f) on recouvre la périphérie de la cupule et le fil de métal biocompatible et non oxydable d'un revêtement isolant biocompatible.

Dans la définition du procédé ci-dessus, on entend par métal biocompatible et non oxydable, un métal biocompatible qui n'est pas oxydé lors du stade e du procédé. Il s'agit de préférence de l'or. D'autres métaux tels que le platine et l'iridium peuvent être également utilisés.

De préférence dans le stade f on recouvre toute la surface extérieure de la cupule d'un revête ment isolant biocompatible et l'on décapite l'extrémité du revêtement de la cupule lors de son utilisation.

L'invention est exposée ci-après plus en détail en se référant aux dessins annexés sur lesquels :

— les Figures 1A à 1H représentent les différentes phases de la fabrication d'une électrode selon l'invention,

— les Figures 2A et 2B représentent les différentes phases de la fixation d'une cosse à l'extrémité du conducteur de l'électrode, et

— la Figure 3 représente une prothèse selon l'invention après implantation.

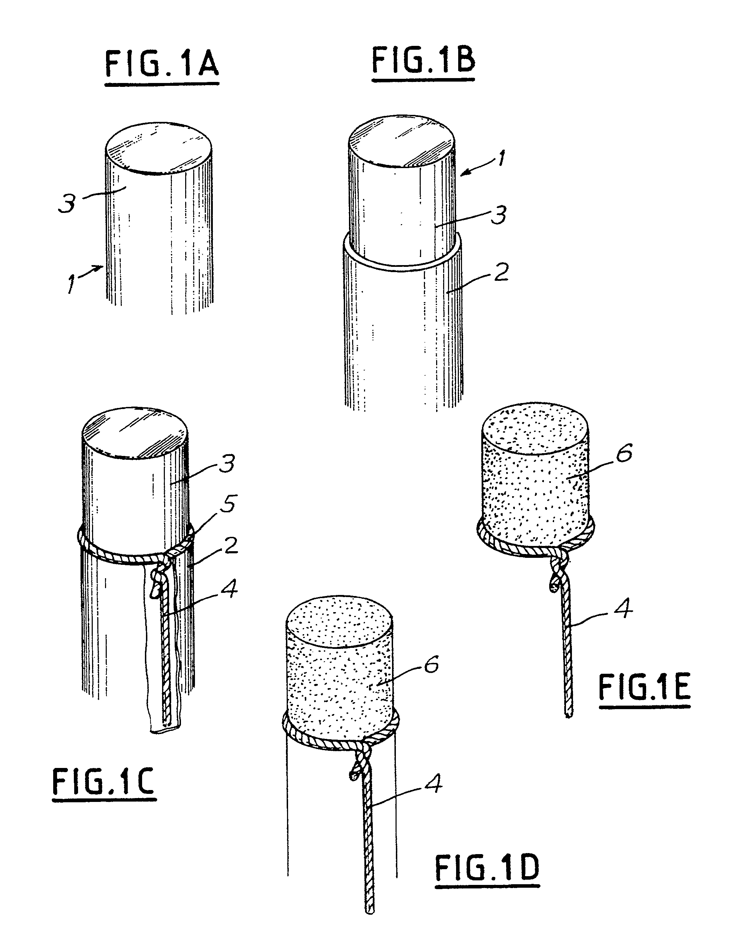

Sur les Figures 1A à 1H on a représenté les différentes phases de la fabrication d'une électrode selon l'invention destinée à être appliquée à l'extrémité d'un élément d'interposition, notamment d'un piston en téflon classiquement utilisé dans une prothèse stapédo-vestibulaire.

Dans le premier stade du procédé on fabrique une matrice cylindrique métallique ayant un diamètre identique à celui du piston prévu pour la mise en place de l'électrode. Cette matrice subit un alésage, un arrasage, un polissage lui donnant la forme et la dimension voulues et un aspect brillant.

Comme représenté sur la Figure 1B, dans le deuxième stade du procédé on protège la partie à épargner lors de l'électroformage ultérieur par une couche de vernis et un tube de silicone 2 en ne laissant à découvert que l'extrémité 3 de la matrice cylindrique.

On fabrique par ailleurs, un fil d'or souple à l'aide de plusieurs brins ayant par exemple chacun 25 microns de diamètre qui sont torsadés ensemble. Ce fil souple est nécessaire pour maintenir la mobilité du piston auquel sera attaché l'électrode. Ce fil d'or 4 est ensuite enfilé sous le tube de silicone 2 et son extrémité 5 est appliquée contre ou autour de l'extrémité 3 de la matrice 1 de façon par exemple à former une boucle autour de cette extrémité 3, comme représenté sur la Figure 1C.

L'ensemble est ensuite soumis à un électroformage par dépôt électrolytique dans un bain de sel d'or non toxique. Le dépôt électrolytique est effectué par exemple jusqu'à ce que l'on obtienne un dépôt de plusieurs dizaines de microns permettant une rigidité et une soudure électrolytique suffisantes. Comme représenté sur la Figure 1D, ce dépôt forme une cupule 6 recouvrant l'extrémité 3 de la matrice 1. Lors du dépôt électrolytique l'extrémité 5 du fil d'or est soudée à la cupule 6. Le procédé décrit dans la publication déjà citée permet de réduire l'épaisseur du dépôt à une faible valeur car la dureté de l'or ainsi déposé est d'environ 250 Vickers. Il est avantageux de réduire au maximum l'épaisseur pour que le diamètre total de l'électrode soit de faible dimension et ne vienne pas, après mise en place, en contact avec le nerf facial très proche de la fenêtre ovale.

On élimine ensuite la matrice métallique par voie chimique en plongeant l'ensemble de la matrice cupule/fil d'or dans de l'acide sulfurique ou tout autre acide permettant la dissolution de la matrice. On obtient ainsi une cupule 6 sur laquelle est fixée le fil d'or, comme représenté sur la Figure 1E.

Comme représenté sur la Figure 1F on insère ensuite un piston de téflon 7 à l'intérieur de la cupule 6, ce piston ayant un diamètre tel qu'il s'introduit avec friction afin qu'il ne puisse pas se séparer lors de la mise en place.

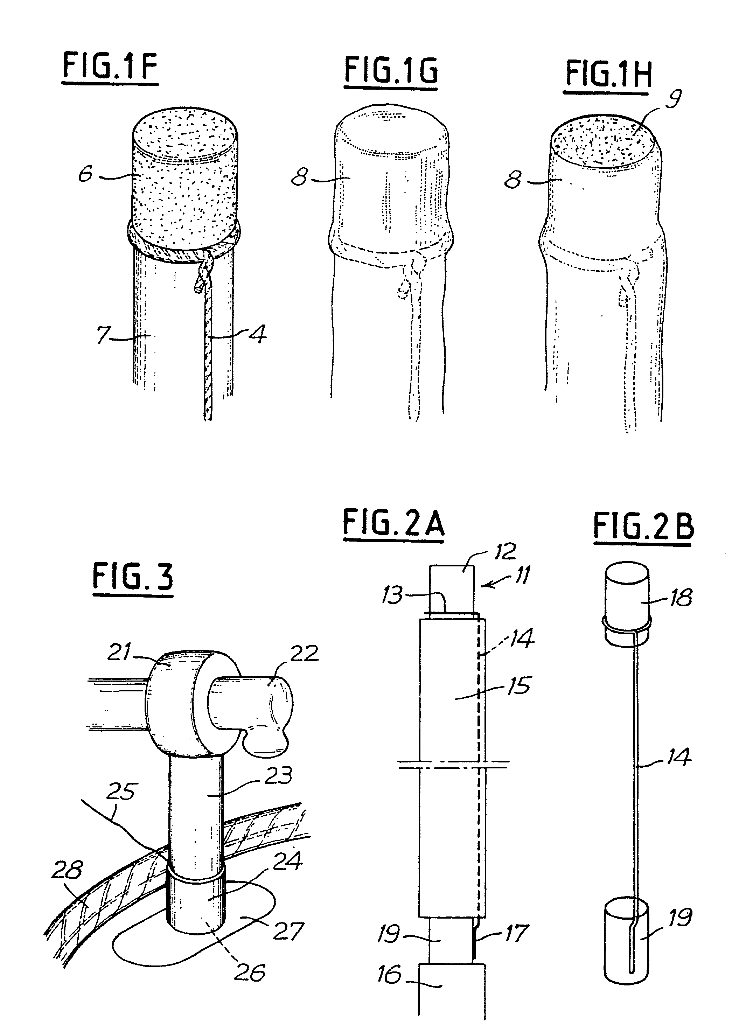

L'ensemble de la cupule 6 et du fil 4 est ensuite immergé dans un bain de silicone biocompatible adhésif dissous dans du trichloréthylène, à plusieurs reprises de façon à obtenir, par dépôts successifs capillaires, un revêtement total ayant une épaisseur suffisante comme représenté sur la Figure 1G. L'ensemble est ensuite laissé à l'air libre pendant 24 heures jusqu'à polymérisation complète et élimination de toute trace de solvant.

L'électrode est maintenant prête pour son implantation. Il suffit lors de l'intervention de décapiter l'extrémité du revêtement de silicone 8 de façon à faire apparaître la surface plane en or 9 qui constitue l'électrode de stimulation électrique, comme représenté sur la Figure 1H.

Dans le procédé selon la présente invention on peut également fabriquer simultanément une cosse de connection comme représenté sur la Figure 2A et la Figure 2B. On utilise à cet effet une matrice 11 de grande longueur dont l'extrémité 12 est entourée par l'extrémité 13 d'un fil d' or 14. L'ensemble de la matrice 12 et du fil d'or 14 est entouré par un manchon 15 isolant qui est interrompu au voisinage de l'autre extrémité de la matrice 11 de façon à laisser apparent une partie 16 de la matrice et une partie 17 correspondante du fil d'or.

L'ensemble est soumis comme précédemment à un électroformage par dépôt électrolytique dans un bain de sel d'or de façon à déposer l'or à la fois sur l'extrémité 12 de la matrice 11 et sur la partie 16 de la matrice 11. Après démontage et élimination par voie chimique de la matrice on obtient une cupule d'or 18 reliée par le fil 14 à une cosse d'or 19.

L'ensemble, après protection des parties internes est ensuite revêtu extérieurement d'un revêtement isolant biocompatible.

On a représenté sur la Figure 3 une prothèse selon l'invention après mise en place. Lors de l'intervention l'étrier est enlevé et à sa place est fixée la prothèse selon l'invention.

La prothèse comprend une tête de fixation 21 qui vient entourer la branche descendante de l'enclume 22. Cette tête de fixation 21 est solidaire d'un piston en téflon 23 à l'extrémité duquel est fixée l'électrode selon l'invention 24 qui est reliée par le fil d'or 25 à une alimentation. L'extrémité 26 qui constitue l'électrode proprement dite est en contact avec la membrane 27 de la fenêtre ovale ou bien un tissu tel qu'une greffe veineuse est insérée entre les deux.

Comme on peut le voir sur la Figure 3 il est nécessaire que le diamètre de l'électrode soit suffisamment petit pour éviter tout contact avec le nerf facial 28. Une autre électrode ou électrode de référence est implantée dans les tissus voisins.

Il est évident que l'élément d'interposition peut non seulement être constitué de téflon mais de tout autre matériau non conducteur biocompatible qui puisse être aisément sectionné lors de l'intervention pour pouvoir l'adapter au patient.

La prothèse selon l'invention peut être non seulement utilisée dans le cas de surdité neurosensorielle complète, mais également dans le traitement des acouphènes et des bourdonnements.

## Revendications

1. Prothèse permettant à la fois une stimulation acoustique et une stimulation électrique de l'oreille interne, caractérisée en ce qu'elle comprend un élément d'interposition (23) dont l'une des extrémités est munie de moyens de fixation (21) à la branche descendante de l'enclume (22) et dont l'autre extrémité, destinée à être appliquée sur la fenêtre ovale, est munie d'une surface d'extrémité conductrice (26) constituant une électrode qui est reliée par un conducteur (25) recouvert d'un revêtement isolant biocompatible à une source de stimulation électrique.

2. Prothèse selon la revendication 1, caractérisée en ce que l'électrode est constituée par une cupule métallique (6) dans laquelle l'extrémité de l'élément d'interposition (23) est insérée et dont la surface périphérique est recouverte d'un revêtement isolant biocompatible, ladite cupule (6) étant soudée audit conducteur (25).

3. Prothèse selon la revendication 2, caractérisée en ce que la cupule est réalisée et est soudée au conducteur par électroformage.

4. Prothèse selon la revendication 2 ou la revendication 3, caractérisée en ce que la cupule et le conducteur sont en or.

5. Prothèse selon la revendication 4, caractérisée en ce que le conducteur est constitué par des fils d'or torsadés.

6. Electrode de stimulation électrique de l'oreille interne destinée à être fixée à l'extrémité d'un élément d'interposition, caractérisée en ce qu'elle est constituée par une cupule (6) métallique dont la surface périphérique est recouverte d'un revêtement isolant biocompatible, ladite cupule étant soudée à un conducteur recouvert d'un revêtement isolant biocompatible.

7. Electrode de stimulation électrique selon la revendication 6, caractérisée en ce qu'elle est réalisée et soudée au conducteur par électroformage.

8. Electrode de stimulation électrique selon la revendication 6 ou la revendication 7, caractérisée en ce que la cupule et le conducteur sont en or.

9. Electrode de stimulation électrique selon la revendication 8, caractérisée en ce que le conducteur est constitué de fils d'or torsadés.

10. Procédé de fabrication d'une électrode selon l'une quelconque des revendications 7 à 9, caractérisé en que

a) on fabrique une matrice métallique du diamètre de l'élément d'interposition sur lequel l'électrode sera fixée,

b) on recouvre par un isolant la matière à l'exception d'une zone d'extrémité,

c) on applique contre la zone d'extrémité non recouverte de la matrice l'extrémité d'un fil de métal biocompatible et non oxydable,

d) on forme électrolytiquement un dépôt de métal biocompatible et non oxydable sur la zone non recouverte de la matrice de façon à former une cupule de métal biocompatible et non oxydable lié à l'extrémité du fil de métal biocompatible et non oxydable,

e) on élimine la matrice métallique par voie chimique et

f) on recouvre la périphérie de la cupule et le fil de métal biocompatible et non oxydable d'un revêtement isolant biocompatible.

11. Procédé selon la revendication 10, caractérisé en ce que dans le stade f on recouvre toute la surface extérieure de la cupule (6) d'un revêtement isolant biocompatible et l'on décapite l'extrémité du revêtement de la cupule lors de son utilisation.

12. Procédé selon la revendication 10 ou la revendication 11, caractérisé en ce que l'on munit l'autre extrémité du fil de métal noble d'une cosse de connexion (19) par électroformage lors du stade d.

## Claims

1. A prosthesis allowing the simultaneous acoustic and electric stimulation of the inner ear, characterized in that it comprises an interposed element (23) one of whose ends has means (21) for attachment to the descending arm of the anvil (22), its other end, which is adapted to be applied to the oval window, having a conductive end surface (26) forming an electrode which is connected via a conductor (25) coated with a biocompatible insulating coating to a source of electric stimulation.

2. A prosthesis according to claim 1, characterized in that the electrode is formed by a metal cupula (6) into which the end of the interposed element (23) is inserted and whose peripheral surface is coated with a biocompatible insulating coating, said cupula (6) being welded to said conductor (25).

3. A prosthesis according to claim 2, characterized in that the cupula is produced and welded to the conductor by electroforming.

4. A prosthesis according to claims 2 or 3, characterized in that the cupula and the conductor are of gold.

5. A prosthesis according to claim 4, characterized in that the conductor is formed by twisted gold wires.

6. An electrode for the electric stimulation of the inner ear, adapted to be attached to the end of an interposed element, characterized in that it is formed by a metal cupula (6) whose peripheral surface is coated with a biocompatible insulating coating, said cupula being welded to a conductor coated with a biocompatible insulating coating.

7. An electrode for electric stimulation according to claim 6, characterized in that it is produced and welded to the conductor by electroforming.

8. An electrode for electric stimulation according to claims 6 or 7, characterized in that the cupula and the conductor are of gold.

9. An electrode for electric stimulation according to claim 8, characterized in that the conductor is formed by twisted gold wires.

10. A process for the production of an electrode according to any of claims 7 to 9, characterized in that

a) a metal matrix is produced having the diameter of the interposed element to which the electrode will be attached,

b) the matrix is coated with an insulating material except for an end zone,

c) the end of the biocompatible, non-oxidizable metal wire is applied against the uncoated end zone of the matrix,

d) a deposit of biocompatible, non-oxidizable metal is so formed electrolytically on the uncoated zone of the matrix as to form a biocompatible, non-oxidizable metal cupula connected to the end of the biocompatible, non-oxidizable metal wire,

e) the metal matrix is eliminated chemically, and

f) the periphery of the cupula and the biocompatible, non-oxidizable metal wire are coated with a biocompatible insulating coating.

11. A process according to claim 10, characterized in that in stage f the whole outer surface of the cupula (6) is coated with a biocompatible insulating coating and the end of the coating of the cupula is cut off when it is used.

12. A process according to claims 10 or 11, characterized in that during the stage d the other end of the precious metal wire is provided with a connecting socket (19) by electroforming.

**Patentansprüche**

1. Prothese, die gleichzeitig eine akustische Stimulation und eine elektrische Stimulation des Innenohres erlaubt, **dadurch gekennzeichnet,** daß sie ein Zwischensetzteil (23) aufweist, bei dem eines seiner Enden mit einer Befestigungseinrichtung (21) an dem absteigenden Ast des Amboß (22) versehen ist, und dessen anderes Ende, das dazu dient, auf das ovale Fenster aufgebracht zu werden, mit einer leitenden Endoberfläche (26) versehen ist, die eine Elektrode bildet, die über einen Leiter (25), der mit einem biokompatiblen isolierenden Überzug bedeckt ist, mit einer elektrischen Erregerquelle verbunden ist.

2. Prothese nach Anspruch 1, **dadurch gekennzeichnet,** daß die Elektrode durch einen metallischen Becher (6) gebildet wird, in dem das Ende des Zwischensetzteils (23) eingeführt ist, und von dem die Außenoberfläche mit einem biokompatiblen isolierenden Überzug bedeckt ist wobei der Becher (6) an den Leiter (25) angeschweißt ist.

3. Prothese nach Anspruch 2, **dadurch gekennzeichnet,** daß der Becher hergestellt und an den Leiter angeschweißt ist durch Elektro- bzw. Galvanoformung.

4. Prothese nach Anspruch 2 oder 3, **dadurch gekennzeichnet,** daß der Becher und der Leiter aus Gold sind.

5. Prothese nach Anspruch 4, **dadurch gekennzeichnet,** daß der Leiter aus verdrillten Goldfäden gebildet ist.

6. Elektrode zur elektrischen Anregung des Innenohrs, die dazu dient, am Ende eines Zwischensetzteils befestigt zu werden, **dadurch gekennzeichnet,** daß sie aus einem metallischen Becher (6) gebildet ist, dessen Außenoberfläche mit einem biokompatible isolierenden Überzug bedeckt ist, wobei der Becher an einen mit einem biokompatiblen isolierenden Überzug bedeckten Leiter angeschweißt ist.

7. Elektrische Erregerelektrode nach Anspruch 6, **dadurch gekennzeichnet,** daß sie durch Elektrobzw. Galvanoformung erzeugt wird und an den Leiter angeschweißt wird.

8. Elektrische Erregerelektrode nach Anspruch 6 oder 7, **dadurch gekennzeichnet,** daß der Becher und der Leiter aus Gold sind.

9. Elektrische Erregerelektrode nach Anspruch 8, **dadurch gekennzeichnet,** daß der Leiter aus verdrillten Goldfäden gebildet ist.

10. Verfahren zur Herstellung einer Elektrode nach einem der Ansprüche 7 bis 9, **dadurch gekennzeichnet,** daß

a) eine metallische Matrix vom Durchmesser des Zwischensetzteils hergestellt wird, auf dem die Elektrode befestigt werden wird,

b) das Material mit Ausnahme einer Endzone mit einem Isolator überzogen wird,

c) das Ende eines biokompatiblen und nicht oxidierbaren Metallfadens auf die nichtbedeckte Endzone der Matrix aufgebracht wird,

d) elektrolytisch eine biokompatible und nichtoxidierbare Metallablagerung auf der nichtbedeckten Zone der Matrix derart ausgebildet wird, daß ein biokompatibler und nichtoxidierbarer Metallbecher gebildet wird, der mit dem Ende des biokompatiblen und nichtoxidierbaren Metallfadens verbunden wird,

e) auf chemischen Wege die metallische Matrix entfernt wird, und

f) der Umfang des Bechers und der biokompatible und nichtoxidierbare Metallfaden mit einem biokompatiblen isolierenden Überzug bedeckt werden.

11. Verfahren nach Anspruch 10, **dadurch**

EP 0 287 430 B1

**gekennzeichnet,** daß in dem Schritt f) die gesamte äußere Oberfläche des Bechers (6) mit einem biokompatiblen isolierenden Überzug bedeckt wird, und das Ende des Überzugs des Bechers bei seiner Verwendung gekappt wird.

12. Verfahren nach Anspruch 10 oder 11, **dadurch gekennzeichnet,** daß das andere Ende des wertvollen Metallfadens mit einer Verbindungshülse (19) durch Elektro- bzw. Galvanoformung beim Schritt d) versehen wird.

FIG.1A

FIG.1B

FIG.1C

FIG.1D

FIG.1E

# FIG.1F

# FIG.1G

# FIG.1H

# FIG.3

# FIG.2A

# FIG.2B